# EUROPEAN PATENT SPECIFICATION

(11) **EP 0 267 049 B1**
(45) Date of publication and mention of the grant of the patent: **23.03.1994**
(21) Application number: 87309848.7
(22) Date of filing: 06.11.1987
(51) Int. Cl.: G01N 33/50, G01N 1/30

(54) **Method of determining endothelial cell coverage of a prosthetic surface**
Verfahren zur Bestimmung der Endothelialzellenbedeckung einer künstlichen Oberfläche
Méthode de détermination du revêtement d'une surface artificielle par des cellules endothéliales

(30) Priority: 06.11.1986 US 927745
(43) Date of publication of application: 11.05.1988
(73) Proprietor: THOMAS JEFFERSON UNIVERSITY, Philadelphia Pennsylvania 19107 (US)
(72) Inventor: Williams, Stuart K., Wilmington, DE (US); Jarrell, Bruce E., Philadelphia, PA (US)
(74) Representative: Baillie, Iain Cameron

(56) References cited:
- GB-A- 2 145 112
- SURGERY, vol. 101, no. 5, May 1987; J.S. ANDERSON et al., pp. 577-586#
- BIOLOGICAL ABSTRACTS, vol. 72, no. 8, 1981, Biological Abstracts Inc., Philadelphia, PA (US); W.E. BURKEL et al., no. 50292#
- PROCEEDINGS OF THE NATL. ACADEMY OF SCIENCES USA, vol. 77, no. 2, February 1980; L.V. JOHNSON et al., pp. 990-994#
- JOURNAL OF CELL BIOLOGY, vol. 100, March 1985, Rockefeller University Press; P. GREENSPAN et al., pp. 965-973#
- SURGERY, vol. 100, no. 2, 1986; B.E. JARRELL et al., pp. 392-399#
- CHEMICAL ABSTRACTS, vol. 101, no. 1, 02 July 1984, Columbus, OH (US); S. BAUGHMAN et al., p. 448, no. 5171v#
- CHEMICAL ABSTRACTS, vol. 90, no. 18, 30 April 1979, Columbus, OH (US); S.G. GHARFEH et al., p. 513, no. 145348n#

## Description

In the specification, footnotes used are to the following references, which are hereby incorporated by reference into the specification.
1. Edwards, W.H. and Mulherin, J.L. The role of graft material in femorotibial bypass grafts Ann. Surg. 191:721, 1980.
2. Herring, M., Gardner, A., Glover, J., Seeding human arterial prosthesis with mechanically derived endothelium. The detrimental effect of smoking, J. Vasc. Surg., 1984, 1 (2), 279-289.
3. Williams, S., Jarrell, B., Friend, L., Radomski, J., Carabasi, R., Koolpe, E., Mueller, S., Thornton, S., Marinucci, T., and Levine, E. Adult human endothelial cell compatibility with prosthetic graft material. J. Surg. Res. 38:618, 1985.
4. Jarrell, B., Williams, S., Solomon, L., Speicher, L., Koolpe, E., Radomski, J., Carabasi, R., Greener, D., and Rosato, F. Use of an endothelial monolayer upon a vascular graft prior to implantation: temperal dynamics and compatibility with the operating room. Ann. Surg, Vol. 203 No. 6, 671-678, 1986.
5. Jarrell, B., Williams, S., Stokes, G., Hubbard, F., Carabasi, R., Koolpe, E.., Greener, D., Pratt, K., Radomski, J., Speicher, L., and Moritz, M. Use of freshly isolated capillary endothelial cells for the immediate establishment of a monolayer on a vascular graft at surgery. Surgery Vo. 100, No. 2, 392-399, 1986.
6. Radomski, J., Jarrell, B., Williams, S., Koolpe, E., Greener, D., and Carabasi, R. Initial adherence of human capillary endothelial cells to Dacron, In Press, J. Surg. Res.
7. Baker, K.S, Williams, S., Jarrell, B., Koolpe, E., and Levine, E. Endothelialization of human collagen surfaces with human adult endothelial cells. Amer. J. Surg. 150:197, 1985.
8. Durand, R., and Olive, P. Cytocoxicity, mutagenicity and DNA damage by Hoechst 33342 J. Histochem. Cytochem. 30:111, 1982.
9. Williams, S., Sasaki, A., Matthews, M., and Wagner, R. Quantitative determination of deoxyribonucleic acid from cells collected on filters. Anal. Biochem. 107:17, 1980.
10. Greenspan, P., Mayer, E., and Fowler, S., Nile Red: a selective fluorescent stain for intracellular lipid deposits. J. Cell. Biol. 100:965, 1985.
11. Johnson, L., Walsh. M., and Chen, L. Localization of mitochondria in living cells with rhodamine 123. P.N.A.S.. U.S.A. 77:990, 1980.
12. Jarrell, B,, Shapiro, S., Williams, S., Carabasi, R., Levine, E., Mueller, S., and Thornton, S. Human adult endothelial cell growth in culture. J. Vasc. Surg. 6:757, 1984.
13. Thronton, S., Mueller, S., and Levine, E. Human endothelial cells: cloning and long-term serial cultivation employing heparin. Science 222:623, 1983.
14. Jaffe, E.A., Nachman, R., Becker, C., and Minick, C. Culture of human endothelial cells derived from umbilical veins. J. Clin. Invest 52:2745, 1973.
15. Arndt-Jovin, D., and Jovin, T. Analysis and sorting of living cells according to deoxyribonucleic acid content. J. Histochem. Cytochem 25:585, 1977.
16. Johnson, L., Summerhayes, I., and Chen, L. Decreased uptake and retention of rhodamine 123 by mitochondria in feline sarcoma virus-transformed Mink cells. Cell, 28:7, 1982.
17. Chen, L., Summerhayes, I., Johnson, L., Walsh, M., Bernal, S., Lampidis, T, Probing mitochondria in living cells with rhodamine 123. Cold Spring Harbor SymPosium Quant. Biol. 46:141, 1982.
18. Ziegler, M. and Davidson, R. Elimination of mitrochondrial elements and improved viability in hybrid cells. Somatic Cell Genetics, 7:73, 1981.
19. Burke, W., Vinter, D.W., Ford, J.W., Kahn, R.H., Graham, L.M., Stanly, J.C. Sequential studies of healing in endothelial seeded vascular prostheses using fluorescence microscopy. J. Surg. Res. 30(4):305-324 (1981).

The replacement of damaged blood vessels with prosthetic vascular grafts has become a feasible surgical option in recent years. While the use of large lumen grafts has met with considerable success, small lumen grafts often become occluded due to the thrombogenic nature of the materials. Attempts have been made to reduce the thrombogenicity of the grafts by seeding with endothelial cells prior to implantation. While this method has proved feasible in animal models, similar attempts using human endothelial cells have not demonstrated improved patency².

A procedure for attaching human endothelial cells to vascular graft material in vitro has been recently developed³. It is now possible to establish an endothelial cell monolayer on graft after a 1 to 2 hour incubation period^{4_7.} Before this technique can be performed in the operating room, however, a simple method to determine the completeness of the seeding process is necessary. Present methods to visualize endothelial cells on graft material involve fixation procedures which irreversibly damage the cells. Accordingly, there remains a need for a relatively rapid, non-destructive, method of determining the degree of confluence of seeded endothelial cells on a prosthetic surface.

In accordance with this invention there is provided a method of determining endothelial cells coverage on a prosthetic surface comprising applying endothelial cells with a dye capable of exhibiting fluorescent emission in a selected wavelength range, said dye being non-toxic and not interfering with growth of said endothelial cells; illuminating said cells with a light to excite said emission to permit observation of the cell coverage of said prosthetic surface; and performing the method without damage to the surface prior to implantation of the surface into a patient, characterized in that said dye is rhodamine 123, said wavelength range is 500-600 nm, and said surface is expanded polytetrafluoroethylene, a polyester, or polyvinyl alcohol.

The present invention, therefore provides a valuable diagnostic method in which the degree of completeness with which a prosthetic graft has been covered with endothelial cells can be determined. After completion of the steps necessary to allow a monolayer of endothelial cells to form, it is very desirable, if not necessary, to know whether a monolayer truly exists before actual implantation of the graft. This quality control step is necessary to avoid implanting a graft that fails to endothelialize and therefore that would be doomed to thrombosis and failure. For example, it is difficult to visualize living endothelial cells on polyester (such as Dacron polyester) or expanded polytetrafluoroethylene ("ePTFE") surfaces because of their opacity at visible light wave lengths and autofluorescence in the 400 to 500 nm wavelength range. It has been found that cell number can be obtained by staining with conventional nuclear dyes, but that method is destructive and gives little information regarding cell spreading or cell-to-cell contact. According to the present invention, therefore, cell visualization is preferably performed using either cytoplasmic dyes or dyes that exhibit fluorescent emission in the 500 to 600 nm wavelength range. Preferably, dyes having both characteristics are used. It is further preferred that the method of the invention be performed on the actual graft itself rather than on a "control" segment of cell-seeded graft material. Accordingly, the dye should be non-toxic and acceptable for intra-arterial use.

In the study leading to the present invention, alternate fluorescent dyes were used to stain human endothelial cells in vitro. These included the bisbenzamide stain Hoechst 33342, which intercalates between adenine and thymidine base pairs in DNA and has been demonstrated to be nontoxic at low concentrations⁸; mithramycin, an anti-tumor antibiotic which intercalates between guanine and cytosine base pairs⁹; nile red, a lipophilic dye specific for cytoplasmic lipid vesicles¹⁰; sulfofluorescein diacetate (SFDA), a cytoplasm-specific dye; and rhodamine 123, a dye specific to mitochondria¹¹. We have found that of these compounds, rhodamine 123 exhibits the most desirable characteristics. Rhodamine 123 emits a bright orange color when excited with 510 nm light. After a brief exposure to this dye, endothelial cells fluoresce orange while the underlying dacron, ePTFE, or polyvinyl alcohol surfaces show little dye uptake or autofluorescence. Rhodamine 123 selectively stains cytoplasmic structures, allowing visualization of the cell margins. It has no permanent effect on endothelial cell growth or attachment properties in-vitro and is nontoxic. The simplicity of the technique makes it compatible with use in the operating room. An endothelial cell seeded graft could be rapidly evaluated for the number of cells present, the degree of cell spreading as well as the quality of the cell to cell interaction prior to graft implantation.

### BRIEF DESCRIPTION OF THE DRAWINGS

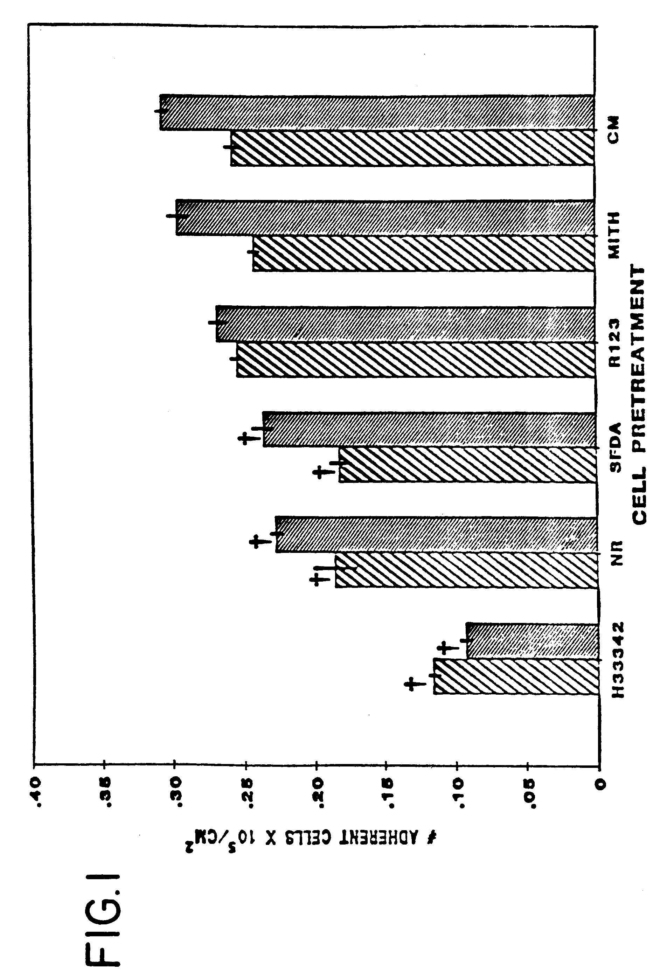

Figure 1: The effects of H33342, Nile red (NR), SFDA, Rhodamine 123 (R123), Mithramycin (Mith) and plain culture medium (CM) on endothelial cell adherence to polystyrene. Human microvessel endothelial cells were incubated for 90 minutes in medium containing 20 ug/ml H33342, 10 ug/ml Nile red, 20 ug/ml SFDA, 10 ug/ml rhodamine 123 or 10 ug/ml mithramycin. After incubation, 1.96 x 104 cells/cm² were seeded onto gelatin coated polystyrene. These labelled cells were left in contact with the surface for 60 or 120 minutes and followed by vigorous washing. The number of remaining adherent cells was determined by brief trypsinization and counting with a Coulter Counter. Each adherence study was performed in triplicate. Results were evaluated by calculation of the mean ± the standard error of the mean and compared using the Student's T-test. Statistical significance was present for p less than 0.05 (†). H33342, Nile Red and SFDA demonstrated a statistically significant decrease in cell adherence at both 60 and 120 minutes when compared to unlabelled cells (CM).

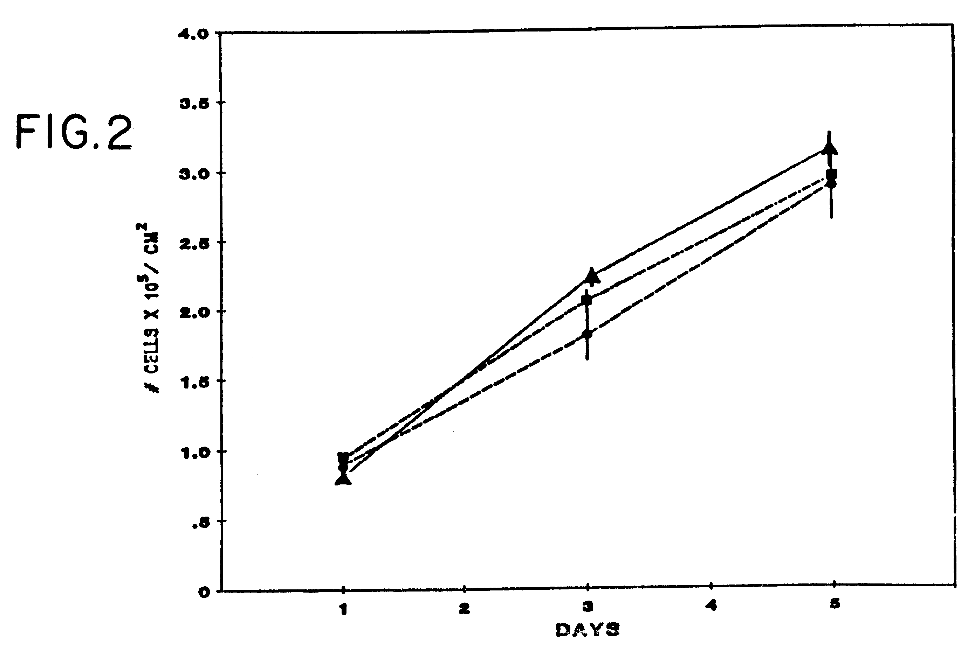

Figure 2: Growth curves demonstrating the effect of pre-treatment with 10 uM rhodamine 123 on human microvessel endothelial cells. Cells were incubated for 90 minutes in one of three media: 1. Culture medium ( ▲ ); 2. Culture medium containing 0.1% DMSO ( ● ); or 3. Culture medium containing ( ■ ) 0.1% DMSO and 10 uM rhodamine 123 ( ■ ). Following incubation, the cells were washed and seeded onto gelatin coated polystyrene at 5 x 10⁴ cells/cm² and maintained in culture medium. Cell number was determined on days 1, 3, and 5 and expressed as the mean ±-standard error of the mean. Multiple regression analysis revealed no statistical difference in cell number. This suggests that pre-labelling with rhodamine 123 has a minimal effect on cell growth.

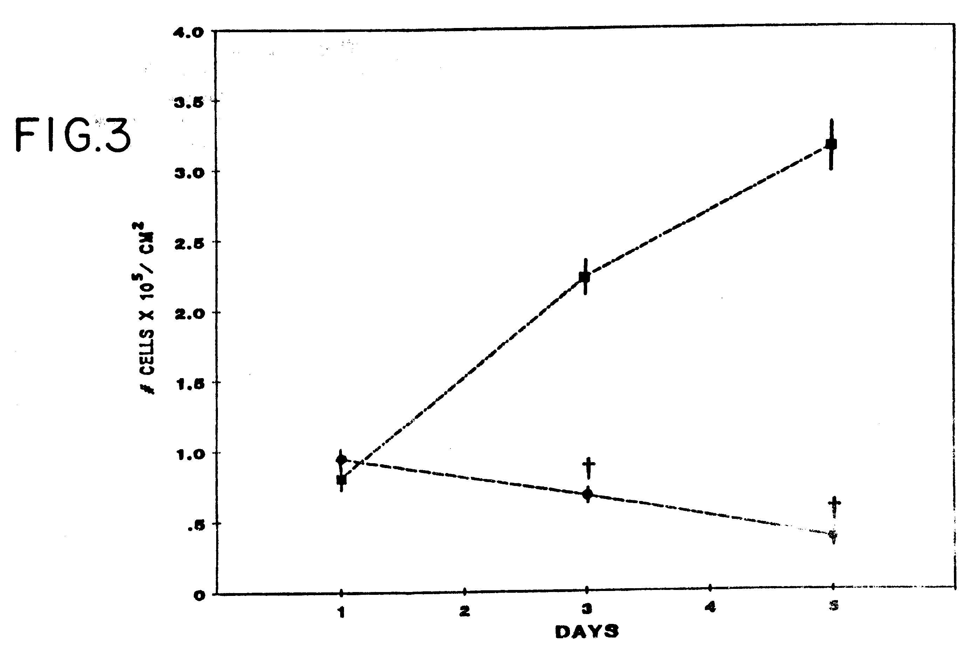

Figure 3: Growth curves demonstrating the effect of culture medium containing rhodamine 123 on human microvessel endothelial cells. Cells were washed and seeded onto gelatin coated polystyrene at 5 x 104 cells/cm² and maintained in culture medium containing 10 uM rhodamine 123 ( ● ) or culture medium containing no rhodamine 123 ( ■ ). Cell number was determined on days 1, 3, and 5 and expressed as the mean ± standard error of the mean. Multiple regression analysis revealed a statistical difference in cell growth between cells grown in rhodamine 123-containing culture medium versus culture medium. Prolonged contact with rhodamine 123 prevents cell growth in culture.

### DETAILED DESCRIPTION OF THE INVENTION

The ability to isolate and grow human adult endothelial cells in culture has allowed an in-depth evaluation of endothelial cell functional properties³. one area of intense investigation has been directed at understanding the variables that affect EC-prosthetic surface interactions³. The optimal conditions for cell adherence and proliferation have not yet been determined. Once these conditions are defined, it may be possible to completely coat the lumen of a vascular graft with endothelial cells in the operating room prior to implantation. This theoretically might minimize thrombus formation and early graft failure. One major obstacle to understanding the process of monolayer formation has been the inability to observe endothelial cells directly upon graft material. Prior to the development of the technique described in this specification, most visualization procedures required permanent fixation of samples at multiple experimental time points to evaluate the effects of different conditions. Permanent fixation not only kills cells but also introduces potential artifacts.

The purpose of the study leading to the present invention was the development of a rapid, easily performed non-toxic method for endothelial cell visualization on dacron, ePTFE, polyvinyl alcohol, and other graft materials. We examined a group of fluorescent dyes which have numerous applications in cell biology ranging from the visualization of cytoplasmic components 10,11 to flow cytometry¹⁵. Our results demonstrated that rhodamine 123 was the most useful for visualizing endothelial cells on woven dacron, ePTFE and PVA. Rhodamine 123 has been used in histology for comparing normal and transformed fibroblasts¹⁶, assessing the efficacy of chemotherapy¹⁷ and selection of cells containing mutations¹⁸. Its specificity for mitrochondria appears to be due to the high negative charge within the mitochondria and the strongly positive charge of rhodamine 123 at physiological pH.

For our purposes, rhodamine 123 has several desirable features. It is readily assimilated into the cells in a concentration-dependent manner and is relatively nontoxic to the cells. While cytostatic when maintained in culture, brief exposure to the dya followed by culture in non-dye containing medium has little demonstrable detrimental effect. The vehicle, 0.1% DMSO, also has an insignificant effect on cells with respect to adherence or growth. This apparent lack of toxicity makes it feasible to treat endothelial cells with rhodamine 123 and then seed them onto dacron, ePTFE or PVA in plain medium without affecting cell adherence or growth. Finally, rhodamine 123 fluoresces at a wavelength where the autofluorescence of dacron, ePTFE and PVA is negligible.

The ability to visualize human endothelial cells on vascular graft material has two major applications. In the laboratory, fluorescent labeling of endothelial cells with rhodamine 123 allows direct observation of the morphological response of living cells to various experimental conditions. As a consequence, the variables affecting endothelial cell adherence and growth on vascular grafts can be evaluated in real time rather than following chemical fixation. Once these conditions are defined, it may be possible to refine current graft seeding techniques.

In the clinical setting, this technique could be used to assess the extent of endothelial cell seeding on vascular grafts in the operating room. We have recently demonstrated that large numbers of endothelial cells can be rapidly harvested from small quantities of human fat⁵. The number of cells obtained is sufficient to allow graft seeding at confluent densities without the need for cell culture. In addition, we have demonstrated that these endothelial cells have the potential to establish a confluent monolayer on certain vascular grafts in less than one hour. One concern with this system is the ability to rapidly assess the completeness of endothelial cell seeding on a graft prior to implantation. One potential method of performing this quality control step is direct fluorescent visualization of the final monolayer using rhodamine 123. This nontoxic fluorescent dye allows rapid visualization of endothelial cells and permits assessment of both the number of cells present and the quality of cell to cell interactions.

### Methods

Isolation of Human Fat Microvessel Endothelial Cells: Human peri-renal and omental fat were obtained from cadaver renal donors. Appropriate institutional review-procedure was followed, Microvessel endothelial cells were isolated by mechanical mincing, digestion with collagenase (Worthington Type I; Worthington Diagnostic Systems, Freehold, N.J.) at a concentration of 4 mg/ml, followed by density gradient centrifugation in 45% Percoll (Pharmacia Fine Chemicals, Piscataway, N.J.)5. The cells were grown in 25 cm² culture flasks which had been pretreated with a 1% gelatin solution. The culture medium consisted of medium 199 with 20% heat-inactivated fetal calf serum, 90 ug/ml porcine heparin, and 20 ug/ml Endothelial cell Growth Factor (ECGF)12,13. The cells were incubated at 37°C in a 5% CO2 atmosphere and at confluence reached a density of 105 cells per cm².
Isolation of Large Vessel Endothelial Cells: Human large blood vessels were cleaned of their surrounding fascia and rinsed with culture medium¹⁴. The ends of the vessel were cannulated and the lumen treated with collagenase (Worthington type I, Worthington Diagnostic Systems, Inc., Freehold, N.J.) to dislodge the endothelial cells from the inner wall. The vessels were then flushed with culture medium to collect the cells. The large vessel cells were cultured under conditions identical to that used for the microvessel endothelial cells.
Preparation of Graft Material: Graft materials made of woven Dacron (Meadox, Medicals, Inc.) and ePTFE (W.L. Gore, Inc.) were immobilized in plastic rings (Beem capsule, Polysciences, Fort Washington, PA) with a 0.5 cm² surface area. Polyvinyl alcohol (PVA), a hydrogel not presently used for vascular grafts, was glued to the end of glass tubing of the same dimensions. Prior to use, the dacron and ePTFE were degreased by washing sequentially with acetone, 8.5% phosphoric acid, 1 N sodium hydroxide and distilled water. The graft material was then dried thoroughly and gas sterilized. The PVA was hydrated in PBS for at least 24 hours prior to use.
Preparation of Platelet Rich Plasma: Fresh human blood was drawn into tube containing sodium citrate. The blood was centrifuged for 6 minutes at 300 xg. The supernatant was combined with calcium chloride to a final concentration of 20 mM. Two hundred ul of this solution were immediately used to coat the dacron and ePTFE at the bottom of each Beem capsule and forced through the graft by means of a micropipette. When clotting was complete, residual clot was removed from the graft by aspiration. PVA underwent no pretreatment other than hydration.
Seeding of Endothelial Cells: Confluent flasks of endothelial cells were treated for several minutes with a solution containing 0.25% trypsin and 0.04% EDTA in buffered Hank's salts. When the cells had lifted from the bottom of the flask, culture medium was added to inactivate the trypsin. Cells were seeded onto the graft at densities up to 105 cells per cm².
Light Microscopy: Seeded grafts were washed with culture medium, then fixed with 95% ethanol for 15 minutes at room temperature. The grafts were washed twice with distilled water, then treated with Gill's hemotoxylin (Fisher Scientific Co., Fairlawn, N.J.) for 2.5 minutes. The grafts were washed twice with distilled water, then exposed to Scott's tap water substitute (Fisher Scientific Co., Fairlawn, N.J.) for one minute. The grafts were washed twice with distilled water, twice with 95% ethanol, then mounted between a glass slide and cover slip for examination by light microscopy.
Application of Fluorescent Dyes: A 20 ug/ml solution of H33342 (Sigma Chemical Co., St. Louis, MO) in Dulbecco's Phosphate Buffered Saline (PBS), pH 7.4 was prepared fresh for each use. A 10 ug/ml solution of mithramycin (Sigma Chemical Co., St. Louis, MO) was prepared by combining 0.5 ml of mithramycin stock solution (0,2 mg/ml in PBS), 0.5 ml of a magnesium chloride solution (300 mM in PBS), and 9 ml of culture medium. Nile red (Eastman Kodak Co., Rochester, New York) was first dissolved in acetone at 1 mg/ml and then diluted 1:100 in PBS to give a final concentration of 10 ug/ml. Sulfofluorescein diacetate (Molecular Probes, Junction City, OR) was used at a concentration of 20 ug/ml in PBS. A 4 mg/ml stock solution of rhodamine 123 (Molecular Probes, Junction City, OR) in DMSO was diluted 1:1000 in culture medium to give a final concentration of 4 ug/ml. All solutions were filtered through Gelman 0.2 micron disposable filters (Gelman Science, Inc., Ann Arbor, MI) for sterilization prior to use.

Endothelial cells were treated with dye in three ways. Seeded Beem capsules were washed once with culture medium, then immersed in dye solution for 30 to 90 minutes at 37°C. Alternatively, cells grown to confluence in culture flasks were treated with trypsin, pelleted by centrifugation for 6 minutes at 300 x g, resuspended in dye solution, and incubated for 30 to 90 minutes at 37°C. These cells were then seeded onto graft material and incubated for varying lengths of time to permit cell adherence. Finally, in the case of rhodamine 123, H33342, and mithramycin, cells were cultured for up to five days at 37°C. in culture medium containing varying concentrations of these compounds, then seeded onto graft material. All endothelial cells, (labelled and control), were then visualized by fluorescence or light microscopy after seeding onto graft material.
Fluorescence Microscopy: Fluorescence microscopy was performed using a Nikon diaphot microscope.
Excitation and emission wavelengths were controlled by specific combinations of excitation and emission barrier filters in a dichroic filter combination (380 nm dichroic mirror for H33342 and sulfofluorescein diacetate; 510 nm dichroic mirror for mithramycin; 550 nm dichroic mirror for Nile red and rhodamine 123).
Adherence Studies: The effects of various dyes on endothelial cell adherence to polystyrene was determined as follows. Cultured microvessel endothelial cells were incubated for 90 minutes at 37°C in one of nine solutions. These were 20 ug/ml H33342 in Dulbecco's PBS; 10 ug/ml mithramycin in complete medium 199; 10 ug/ml Nile red and 1% acetone in Dulbecco's PBS; 20 ug/ml SFDA in Dulbecco's PBS; and 4 ug/ml (10 uM) rhodamine 123 and 0.1% DMSO in complete medium 199. As controls, cells were also incubated in plain complete medium 199; plain Dulbecco's PBS; Dulbecco's PBS containing 1% acetone; and complete medium 199 containing 0.1% DMSO. The cells were washed twice by centri- fugaton for 6 minutes at 300 x g, resuspended in plain complete medium 199 and seeded in triplicate in gelatin-coated 24 well plates at a density of 1.96 x 104 cells/cm². After 60 and 120 minutes, the medium was aspirated, 0.5 ml trypsin solution added to each well and the cells incubated for 10 minutes at 37°C. 0.2 ml of the resultant cell suspension was then added to 9.8 ml Isoton and the number of adherent cells per well determined using a Coulter counter.
Growth Curve Determination: Cultured peri-renal fat microvessel endothelial cells were incubated for 90 minutes in culture medium containing 10 uM rhodamine 123 and 0.1% DMSO, culture medium containing 0.1% DMSO alone, or plain culture medium. The cells were washed by centrifugation for 6 minutes at 300 x g and seeded in 24 well gelatinized polystyrene culture plates at an initial density of 0.5 x 105 cells per cm². The cells were grown in plain culture medium, or culture medium plus 0.1% DMSO and 10 uM rhodamine 123. At designated time points the medium was aspirated, the cells washed once with medium and treated with 0.5 ml trypsin for 10 minutes at 37°C. 0.2 ml of this suspension was added to 9.8 ml of Isoton solution and the cells counted in a Coulter counter.

### Statistics:

EC cell number in each study was determined by counting cells with a Coulter counter for three replicate experiments and expressed as cell number ± standard error of the mean. For adherence studies, experimental values were compared to control samples using the Student's t-test. Growth curve analysis was performed using linear regression analysis. Statistical significance was chosen at p less than 0.05.

### Results:

### 1. Staining Properties

Experiments involving H33342 (20 ug/ml) and mithramycin (10 ug/ml) demonstrated that incubating human aorta, iliac vein or microvessel endothelial cells for 90 minutes at 37°C. resulted in successful labeling of the cell nucleus. Similarly, Nile red rhodamine 123 and SFDA at a concentration of 20 ug/ml were effective at fluorescently labeling the cell cytoplasm.

A serious disadvantage of H33342, mithramycin, Nile Red, and SFDA was that the wavelengths required to visualize these dyes were associated with significant autofluorescence of the dacron and ePTFE graft material. While andothelial cells treated with these four dyes could be visualized on the grafts, it was often difficult to distinguish cells from the background. A second difficultly encountered with these dyes was the high degree of non- specific incorporation of the dyes into the graft material. In the absence of cells, graft material treated with any of these four dyes for 90 minutes at 37°C. showed discrete areas of fluorescence that could be mistaken for cells. For these reasons, H33342, mithramycin, sulfofluorescein diacetate, and Nile red were unacceptable for our purposes.

To examine the effects of rhodamine 123, human microvessel endothelial cells were incubated for 90 minutes at 37°C. in culture medium containing 1, 5, 10, 20 and 100 uM rhodamine 123 (0.4, 2.0, 4.0, 8.0, 40.0 ug/ml) and the appropriate dilution of DMSO and examined by fluorescent microscopy. There was a concentration-dependent increase in intensity of fluorescence. Cells treated with 1 uM rhodamine 123 demonstrated faint fluorescence while incubation in 100 uM solution produced strong fluorescence.

When cells were labelled with rhodamine 123 (4 ug/ml) followed by incubation on either dacron, ePTFE or PVA surfaces, excellent cytoplasmic visualization was obtained with minimal surface autofluorescence. Excitation and emission wavelength were 510 nm and 590 nm, respectively, in all cases.

### 2. Adherence effects

The adherence of microvessel endothelial cells to polystyrene was examined for each of the five fluorescent dyes. Cells were preincubated with each dye at concentrations listed in the methods for 90 minutes. Following labelling, the cells were seeded onto polystyrene at 1.96 x 104 cells/cm² and incubated for 1 and 2 hours. At that time, the surface was vigorously washed twice. The cells remaining on the surface were counted following brief trypsinization. The number of remaining cells is shown in Figure 1. When compared to unlabelled control cells, H33342 Nile red and SFDA demonstrated a significant effect on subsequent cell adherence a measured at 60 and 120 minutes. Mithramycin and rhodamine 123 had no effect on cellular adherence.

### 3. Growth effects after labelling with rhodamine 123

Since rhodamine 123 allowed optimal visualization of endothelial cells on graft surfaces, the effect of labelling the cells prior to growth in culture was examined. Endothelial cells were preincubated with culture medium, medium containing 0.1% DMSO or medium containing 0.1% DMSO and 10 uM rhodamine 123. Following preincubation and washing, the cells were seeded at an initial concentration of 0.5 x 105 cells per cm² and placed in culture. Cell number was determined on days 1, 3, and 5. The growth curves in Figure 2 show little difference in subsequent cell growth in culture medium 199 between prelabelled and unlabelled cells.

### 4. Growth curves in medium containing rhodamine 123

Since prelabelling of cells with rhodamine 123 had little effect on subsequent cell growth, the effect of continued rhodamine 123 in the culture medium was examined. Cells were seeded at 0.5 x 105 cells/cm² onto polystyrene and placed in culture. Culture medium for this growth curve study contained rhodamine 123 throughout the time of the study. Rhodamine 123 maintained in the culture medium prevented cell growth in culture (See figure 3).

## Claims

1. The method of determining endothelial cell coverage on a prosthetic surface comprising applying endothelial cells to said surface; staining said endothelial cells with a dye capable of exhibiting fluorescent emission in a selected wavelength range, said dye being non-toxic and not interfering with growth of said endothelial cells; illuminating said cells with a light to excite said emission to permit observation of the cell coverage of said prosthetic surface; and performing the method without damage to the surface prior to implantation of the surface into a patient, characterized in that said dye is rhodamine 123, said wavelength range is 500-600 nm, and said surface is expanded polytetrafluoroethylene, a polyester, or polyvinyl alcohol.

2. The method of claim 1, characterized in that said surface does not autofluoresce in said selected wavelength range.

3. The method of claim 1, characterized in that said surface is a vascular graft, the wall of which is at least translucent to said emission, and wherein said applying step is performed on the interior surface of said wall.

4. The method of claim 3, characterized in that said graft forms a closed structure with said applied endothelial cells.

## Patentansprüche

1. Verfahren zur Bestimmung der Endothelzellenbedeckung auf einer prothetischen Oberfläche, umfassend Aufbringen von Endothelzellen auf die Oberfläche; Färben der Endothelzellen mit einem Farbstoff, der Fluoreszenzstrahlung in einem ausgewählten Wellenlängenbereich emittieren kann, welcher Farbstoff nichttoxisch ist und das Wachstum der Endothelzellen nicht stört; Beleuchten der Endothelzellen mit einem Licht zum Anregen der Emission, um die Beobachtung der Zellenbedeckung der prothetischen Oberfläche zu ermöglichen; und Durchführen des Verfahrens Ohne Schädigung der Oberfläche vor der Implantation der Oberfläche in einen Patienten,
dadurch gekennzeichnet, daß der Farbstoff Rhodamin 123 ist, der Wellenlängenbereich 500 ... 600 nm beträgt und die Oberfläche geschäumtes Polytetrafluorethylen, ein Polyester oder Polyvinylalkohol ist.

2. Verfahren nach Anspruch 1, dadurch gekennzeichnet, daß die Oberfläche in dem ausgewählten Wellenlängenbereich keine Autofluoreszenz zeigt.

3. Verfahren nach Anspruch 1, dadurch gekennzeichnet, daß die Oberfläche ein Gefäßtransplantat ist, dessen Wand mindestens für die genannte Emission durchlässig ist, und bei welchem der Schritt des Auftragens an der Innenseite der Wand durchgeführt wird.

4. Verfahren nach Anspruch 3, dadurch gekennzeichnet, daß das Transplantat mit den aufgetragenen Endothelzellen eine geschlossene Struktur bildet.

## Revendications

1. Méthode pour vérifier le recouvrement d'une surface prothétique par des cellules endothéliales comprenant l'application de cellules endothéliales sur ladite surface; la coloration desdites cellules endothéliales avec un colorant capable de produire une émission fluorescente dans un intervalle de longueurs d'onde choisi, ledit colorant étant non toxique et ne gênant pas la croissance desdites cellules endothéliales; l'illumination desdites cellules avec une lumière qui excite ladite émission pour permettre l'observation du recouvrement par les cellules de ladite surface prothétique; et la mise en oeuvre de la méthode sans lésion de la surface avant l'implantation de la surface chez un patient, caractérisée en ce que ledit colorant est la rhodamine 123, ledit intervalle de longueurs d'onde est de 500-600 nm et ladite surface est du polytétrafluoroéthylène expansé, un polyester, ou du poly(alcool vinylique).

2. Méthode selon la revendication 1, caractérisée en ce que ladite surface ne produit pas de phénomène d'autofluorescence dans ledit intervalle de longueurs d'onde choisi.

3. Méthode selon la revendication 1, caractérisée en ce que ladite surface est une greffe vasculaire dont la paroi est au moins translucide à ladite émission et dans laquelle ladite étape d'application est effectuée sur la surface interne de ladite paroi.

4. Méthode selon la revendication 3, caractérisée en ce que ladite greffe forme une structure fermée avec lesdites cellules endothéliales appliquées.
